(19) Europäisches Patentamt
European Patent Office
Office européen des brevets

(11) **EP 4 169 895 A1**

(12) **EUROPEAN PATENT APPLICATION**

(43) Date of publication:
**26.04.2023 Bulletin 2023/17**

(21) Application number: **21315225.9**

(22) Date of filing: **21.10.2021**

(51) International Patent Classification (IPC):
*C07C 1/32* (2006.01)  *C07C 11/04* (2006.01)
*C07C 11/06* (2006.01)  *B01J 32/00* (2006.01)
*B01J 29/70* (2006.01)  *C07C 319/02* (2006.01)

(52) Cooperative Patent Classification (CPC):
(C-Sets available)
**C07C 1/322; B01J 21/08; B01J 23/28;
B01J 29/7015; B01J 29/783; B01J 35/002;
B01J 35/1004; B01J 35/1033; B01J 37/0009;
B01J 37/0018; B01J 37/0201; B01J 37/088;
C07C 319/02**                              (Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **TotalEnergies OneTech
92400 Courbevoie (FR)**

(72) Inventors:
• **Veryasov, Gleb
1400 Nivelles (BE)**
• **Nesterenko, Nikolai
1402 Nivelles (Thines) (BE)**

(74) Representative: **Mellet, Valérie Martine
Patent 42
5, rue Dicks
4081 Esch-sur-Alzette (LU)**

(54) **PROCESS AND CATALYST FOR CONVERSION OF CARBON DISULPHIDE INTO C2-C3 OLEFINS**

(57)    The disclosure provides for a process for converting $CS_2$ into C2-C3 olefins, said process comprising (a) providing a gaseous feed stream comprising at least 10 vol. % of $CS_2$; (b) performing a hydrogenation reaction to obtain a first effluent stream (5). comprising methanethiol and hydrogen sulphide; (c) optionally performing a pre-treatment of the first effluent stream (5) and recovering a second effluent stream (9) containing methanethiol and dimethyl sulphide (DMS); (e) providing a catalyst composition; (f) contacting the first and/or second effluent stream with said catalyst composition to convert at least part of said methanethiol and DMS if any; (g) recovering a product stream comprising C2-C3 olefins and unconverted methanethiol and DMS if any. The process is remarkable in that said catalyst composition comprises one or more zeolites with a plurality of pores with a shape of an 8-member ring and with a Si/Al atomic ratio of at most 50.

Figure 1

(52) Cooperative Patent Classification (CPC): (Cont.)

C-Sets
**C07C 1/322, C07C 11/04;**
**C07C 1/322, C07C 11/06;**
**C07C 319/02, C07C 321/04**

**Description**

**Technical field**

The present disclosure relates to a process for converting carbon disulphide ($CS_2$) into C2-C3 olefins.

**Technical background**

In 1985 and 1969, three patents from Mobil Oil (U.S. Pat. Nos. 4,543,434, 4,822,938, 4,864,074) described processes for the conversion of methane to hydrocarbons of higher molecular weight using intermediate compounds comprising sulphur. In these documents, the methane is converted to $CS_2/H_2S$ by oxidation with sulphur (similar to the Folkins process) and then the $CS_2$ is hydrogenated to higher hydrocarbons on a suitable zeolitic catalyst, e.g. ZSM-5. Without an example being given, the description of these documents mentions that the $CS_2$ is first of all converted to $CH_3SH$ and that the latter is the final intermediate in the direction of the hydrocarbons. It is also mentioned that dimethyl sulphide (DMS) could be coproduced during the reaction between the $CS_2$ and the hydrogen.

The reactions for the hydrogenation of $CS_2$ to give methyl mercaptan and then hydrocarbon can be represented diagrammatically as follows:

$$CS_2 + 3H_2 \rightarrow CH_3SH + H_2S \qquad (1)$$

$$nCH_3SH \rightarrow (CH_2)_n + nH_2S \qquad (2)$$

The formation of DMS could be a side reaction of intermolecular $H_2S$ removal from $CH_3SH$, which is an equilibrium reaction:

$$2CH_3SH \rightarrow (CH_3)_2S + H_2S \qquad (3)$$

The other by-products in this reaction could be methane, which might originate from hydrogenolysis or pyrolysis of $CH_3SH$, and other compounds produced due to interaction of the molecules forming in the reaction and/or present in the feed.

The publication by V. Hulea dating 2013 (App. Cat. B., 144, 2014, 547-553) describes the conversion of $CH_3SH$ and its analogue, $CH_3OH$, to hydrocarbons over zeolitic catalysts. Utilization of proper catalyst, e.g., H-ZSM-5, allows producing C1-C3 hydrocarbons and aromatics with conversions of $CH_3SH$ up to 95%. The note should be given that some DMS and coke are also formed in the reaction.

The $CS_2$ hydrogenation reaction aiming at the formation of said intermediate, $CH_3SH$, has been intensively studied and there are patent publications and literature papers available today on the subject.

For example, the U.S. Pat. No. 3,488,739 (The Sun Oil Company, 1970) describes a process for the preparation of methyl mercaptan and dimethyl sulphide from $CS_2$ and hydrogen using a hydrogenation catalyst comprising a mixture of nickel and molybdenum oxides on alumina. At 204 °C, the $CS_2$ conversion is almost complete and the molar selectivities are 33% for $CH_3SH$ and 67% for DMS, with traces of methane. At 177 °C, the $CS_2$ conversion falls to 76% and the molar ratio of the $CH_3SH$/DMS selectivities is 50/50.

The U.S. Pat.' No. 3,880,933 (Phillips, 1975) describes a process for the catalytic hydrogenation of $CS_2$ to give methyl mercaptan in the presence of hydrogen sulphide ($H_2S$). The catalyst used is a mixture of cobalt and molybdenum oxides on alumina. The reaction temperature mentioned is between 230 °C and 260 °C, the reaction pressure between 11.9 and 12.6 bar and the $H_2/CS_2$ molar ratio between 2.75 and 3.5 (3 being the stoichiometric ratio for the production of $CH_3SH$). On using a reasonable amount of $H_2S$ present at the start (H2S/CS2 molar ratio 3), the best result gives a degree of conversion of $CS_2$ of 69% and a molar selectivity for $CS_2$ of 65% and for DMS of 35%, which calculations are carried out starting from the molar compositions shown in the patent.

The international patent application WO 2004/043883 (Georgia Pacific) for its part describes a process for the catalytic conversion of carbon disulphide to methyl mercaptan in the presence of hydrogen. The catalyst can be $V_2O_5$, $Re_2O_7$ or MnO, supported on a substrate selected from $CeO_2$, $ZrO_2$, $TiO_2$, Nb2O5, $Al_2O_3$, $SiO_2$, $Ta_2O_5$ or $SnO_2$, or mixtures of these. It is mentioned that with high conversion of $CS_2$ and high selectivity to $CH_3SH$, the formation of by-products, such as $CH_4$, $C_2H_6$, also occurs.

The publication by L. Shum dating from 1985 (Int. J. Chem. Kinet., 17, 1985, 749-761) describes the thermal decomposition of DMS. In absence of catalyst at 730 K (~457 °C), DMS could be ruptured to give $CH_4$, $C_2H_4$, $H_2S$, $CS_2$ and $C_2H_5SH$.

There is however still a need for a selective process for the conversion of compounds such as $CS_2$ into valuable hydrocarbons such as olefins.

An objective of the present disclosure is to provide a reaction for the catalytic hydrogenation of carbon disulphide to give higher hydrocarbons, namely C2-C3 olefins, and to provide a catalyst composition for this reaction.

Another objective of the disclosure is to provide such a reaction that produces only very little or no by-products, that is say which provides high degrees of conversion, and high selectivity for a hydrogenated product, in particular C2-C3 olefins, in other words, a process which exhibits enhanced performances in terms of yield, conversion, selectivity and productive output.

**Summary of the disclosure**

According to a first aspect, the disclosure provides for a process for converting carbon disulphide into C2-C3 olefins, said process comprising the following steps:

a) providing a gaseous feed stream comprising at least 10 vol. % of carbon disulphide;
b) performing a hydrogenation reaction to obtain a first effluent stream comprising methanethiol ($CH_3SH$) and hydrogen sulphide ($H_2S$);
c) optionally performing a pre-treatment of at least a part of the first effluent stream to convert at least a part of the methanethiol ($CH_3SH$) into dimethyl sulphide (DMS) and recovering a second effluent stream containing methanethiol ($CH_3SH$) and dimethyl sulphide (DMS);
d) optionally, removing of at least a part of $H_2S$ from the first effluent stream and/or the second effluent stream when step (c) is carried out;
e) providing a catalyst composition;
f) contacting the first effluent stream and/or the second effluent stream when step (c) is carried out, with said catalyst composition at a temperature ranging from 100°C to 700°C, at a pressure ranging from 0.2 MPa to 20 MPa, to convert at least part of said methanethiol and dimethyl sulphide if any into C2-C3 olefins;
g) recovering a product stream comprising C2-C3 olefins and unconverted methanethiol ($CH_3SH$) and dimethyl sulphide (DMS) if any;

remarkable in that said catalyst composition provided in step e) comprises one or more zeolites with a plurality of pores with a shape of an 8-member ring and in that the one or more zeolites having a Si/Al atomic ratio of at most 50 as determined by inductively coupled plasma optical emission spectrometry.

Surprisingly, the inventors have found that starting from carbon disulphide a high selectivity to C2-C3 olefins, a high conversion, and subsequently a high yield, can be achieved with the use of a catalyst composition comprising one or more small-pore zeolites (i.e., having 8-member ring pores size) having a Si/Al ratio of at most 50 as determined by inductively coupled plasma optical emission spectrometry.

According to the disclosure, the C2-C3 olefins are ethylene and propylene,

In an embodiment, the one or more zeolites have a Si/Al atomic ratio ranging from 2 to 25, or from 2 to 17, as determined by inductively coupled plasma optical emission spectrometry (corresponding to a SAR 4 to 34); preferably from 3 to 17; more preferably from 5 to 16; even more preferably from 8 to 15; and most preferably from 10 to 15.

In an embodiment, the step (f) of contacting at least the first effluent stream comprising methanethiol and hydrogen sulphide is carried out with the catalyst composition at a temperature ranging from 100°C to 700°C, at a pressure ranging from 0.2 MPa to 20 MPa, to convert at least part of said methanethiol into C2-C3 olefins, wherein the one or more zeolites of said catalyst composition have a Si/Al atomic ratio of at most 15. This allows enhancing selectivity to propylene.

In an embodiment, the one or more zeolites are selected from the group of AEI, AFX, CHA, DDR, ERI, EAB, GIS, KFI, LEV, LTA, RHO, PAU, MWF and RTH families, and any mixture thereof. For example, the one or more zeolites are selected from the group of AEI, CHA, DDR, ERI, KFI, and LEV families, and any mixture thereof. For example, the one or more zeolites are or comprise AEI and/or CHA families.

For example, the one or more zeolites are or comprise SSZ-39 from the AEI family.

For example, the one or more zeolites are or comprise SSZ-13 from the CHA family.

In an embodiment, at least one zeolite, or the one or more zeolites contain at least one metal M being a transition metal and/or a post-transition metal with an atomic ratio Si/(Al+ M) ranging from 2 to 25, or from 2 to 17, as determined by inductively coupled plasma optical emission spectrometry; preferably from 3 to 17; more preferably from 5 to 16; even more preferably from 8 to 15; and most preferably from 10 to 15.

In an embodiment, the step (f) of contacting at least the first effluent stream comprising methanethiol and hydrogen sulphide is carried out with the catalyst composition at a temperature ranging from 100°C to 700°C, at a pressure ranging from 0.2 MPa to 20 MPa, to convert at least part of said methanethiol into C2-C3 olefins, wherein the one or more zeolites of said catalyst composition have a Si/(Al+M) atomic ratio of at most 15. This allows enhancing selectivity to propylene.

In an embodiment, at least one zeolite, or the one or more zeolites contain at least one metal M being a transition metal and/or a post-transition metal wherein the transition metal is selected from Fe, W, V, Mo, Zr, Ag, Ni, Cu, Ti, Zn and any mixture thereof and the post-transition metal is selected from Sn and/or In; preferably, the metal M is selected from Fe, W, V, Mo, Sn, Zr, Ag, Ni, Cu, Ti and any mixture thereof; more preferably, the metal M is selected from Fe, W, V, Mo, Sn, Zr, Ag and any mixture thereof; even more preferably, the metal M is selected from Fe, W, V, Mo, Sn, and any mixture thereof; most preferably, the metal M is selected from V, Mo and any mixture thereof. For example, the metal M is Mo.

In an embodiment, the one or more zeolites are present in the catalyst composition at a content ranging from 25 wt. % to 95 wt. % based on the total weight of said catalyst composition; preferably ranging from 25 to 90 wt.%; more preferably, ranging from 35 to 88 wt.%; even more preferably ranging from 40 to 85 wt.%; most preferably ranging from 50 to 82 wt.%; even most preferably ranging from 60 to 80 wt.%; for example, ranging from 70 to 85 wt.% or ranging from 50 to 90 wt.%.

In an embodiment, the catalyst composition further comprises a binder. With preference, one or more of the following can be used to further define the binder:

- The binder is selected from silica, clays, alumina phosphates, calcium phosphates, magnesium phosphates, mullite and any mixture thereof.
- The binder is or comprises silica.
- The binder is present in an amount of at least 5 wt.% or of at least 10 wt.% as based on the total weight of the catalyst composition, preferably of at least 20 wt.%, most preferably of at least 30 wt.%, even more preferably of at least 40 wt.% and most preferably of at least 50 wt.%.
- The binder is present in an amount ranging from 5 wt.% to 75 wt.% based on the total weight of said catalyst composition; preferably ranging from 10 to 75 wt.%; more preferably, ranging from 12 to 65 wt.%; even more preferably ranging from 15 to 60 wt.%; most preferably ranging from 18 to 50 wt.%; even most preferably ranging from 20 to 40 wt.%; for example, ranging from 15 to 30 wt.% or ranging from 10 to 50 wt.%.

For example, the process further comprises a step of activation of the catalyst composition that is performed before said step (f) of contacting the first effluent stream and/or the second effluent stream. For example, the step of activation comprises calcinating the catalyst composition; with preference, the catalyst composition is calcined at a temperature ranging from 400°C 800°C for a period ranging from 1 h to 24h.

In an embodiment, the gaseous feed stream of step (a) contains at least 15 vol.% of carbon disulphide based on the total molar content of the gaseous feed stream, preferably at least 25 vol.%, more preferably at least 35 vol.%.

In an embodiment, the carbon disulphide is present in the gaseous feed stream of step (a) at a concentration of 100 vol % based on the total volume content of the gaseous feed stream; preferably, of at most 95 vol %; most preferably of at most 75 vol%.

In an embodiment, the gaseous feed stream of step (a) comprises one or more diluents, wherein the one or more diluents are selected from hydrogen sulphide, steam, methane, ethane, propane, butane, carbon dioxide, carbon monoxide, nitrogen, and any mixture thereof. For example, the one or more diluents are selected from steam, methane, ethane, propane, butane, carbon dioxide, carbon monoxide, nitrogen, and any mixture thereof.

In an embodiment, the gaseous feed stream of step (a) comprises one or more selected from methanethiol, $CH_3SH$; ethanethiol, $C_2H_5SH$; 1-propanethiol, $C_3H_7SH$; 2-propanethiol, $CH_3CH(SH)CH_3$; allyl mercaptan; $CH_2=CHCH_2SH$, butanethiol, $C_4H_9SH$; tert-butyl mercaptan, $(CH_3)_3CSH$; pentanethiols, $C_5H_{11}SH$; or any mixture thereof.

In an embodiment, said gaseous feed stream of step (a) further comprises from at least 5 vol.% to at most 25 vol.% of $H_2S$, preferably from 10 vol.% to at most 20 vol.%.

In an embodiment, said gaseous feed stream of step (a) further comprises from at least 5 vol.% to at most 25 vol.% of COS, preferably from 10 vol.% to at most 20 vol.%.

In an embodiment, the one or more diluents are present in the gaseous feed stream of step (a) at a concentration of at most 90 vol % based on the total volume content of the gaseous feed stream of step (a); preferably, of at most 75 vol %; most preferably of at most 65 vol%.

In an embodiment, the process further comprises a step of recycling a part of the C2-C3 olefins recovered at step (g) in the first effluent stream and/or the second effluent stream when step (c) is carried out. For example, the content of the recycled the C2-C3 olefins in the first effluent stream and/or the second effluent stream when step (c) is carried out is at most 10 vol.% based on the total volume of the first effluent stream and/or the second effluent stream when step (c) is carried out; preferably, at most 8 vol.%; more preferably at most 5 vol.% and even more preferably at most 3 vol.%.

For example, the content of the recycled C2-C3 olefins in the first effluent stream and/or the second effluent stream when step (c) is carried out is at least 0.5 vol.% based on the total volume of the first effluent stream and/or the second effluent stream when step (c) is carried out; preferably, at least 1.0 vol.%; more preferably at least 1.5 vol.% and even more preferably at least 2.0 vol.%.

For example, step (f) is performed at a temperature ranging from 200°C to 600°C; preferably ranging from 250°C to 550°C, more preferably, ranging from 300°C to 500°C; even more preferably from 320 °C to 480°C; most preferably to 350 °C to 450°C; and even most preferably from 370°C to 420°C.

In an embodiment, the process further comprises a step of recycling the unconverted carbon disulphide back into step (f).

In an embodiment, step (f) of contacting the first effluent stream and/or the second effluent stream when step (c) is carried out with the catalyst composition is followed by a step (g) of recovering a product stream comprising C2-C3 olefins and unconverted methanethiol and dimethyl sulphide if any; wherein the selectivity to C2- C3 olefins is of at least 25 %; preferably, of at least 30 %; more preferably, of at least 35 %.

According to a second aspect, the disclosure provides for an installation to conduct the process for converting carbon disulphide into C2-C3 olefins according to the process defined in the first aspect, the installation is remarkable in that it comprises:

- a hydrogenation unit;
- optionally, a pre-treatment unit downstream of said hydrogenation unit;
- a conversion unit downstream of the hydrogenation unit or of the pre-treatment unit when said pre-treatment unit is set up, the conversion unit comprising a reactor with a catalyst composition, the reactor having an inlet and an outlet, wherein the catalyst composition comprises one or more zeolites with a plurality of pores with a shape of an 8-member ring and a Si/Al atomic ratio of at most 50 as determined by inductively coupled plasma optical emission spectrometry;
- optionally, a first desulphurisation unit downstream of said hydrogenation unit and upstream of said conversion unit, and/or a second desulphurisation unit downstream of said pre-treatment unit when said pre-treatment unit is set up and upstream of said conversion unit;
- a line to convey a gaseous feed stream comprising the carbon disulphide to the hydrogenation unit;
- a line to convey a first effluent stream exiting the hydrogenation unit to the inlet of the reactor within the conversion unit;
- when the installation comprises a pre-treatment unit, a line to convey said first effluent stream exiting the hydrogenation unit to the pre-treatment unit and a line to convey a second effluent stream exiting the pre-treatment unit to the inlet of the reactor within the conversion unit;
- a first separation unit placed downstream of the conversion unit and configured to separate the unreacted methanethiol and dimethyl sulphide if any and a line to recycle the unreacted methanethiol and dimethyl sulphide if any at the inlet of the reactor within the conversion unit;
- optionally, a second separation unit to recover a part of the olefins produced in the reactor and a line to recycle part of the olefins recovered at the inlet of the reactor.

According to a third aspect, the disclosure provides for the use of a catalyst composition in a process for converting carbon disulphide into C2-C3 olefins remarkable in that the catalyst composition comprises one or more zeolites with a plurality of pores with a shape of an 8-member ring and in that the one or more zeolites have a Si/Al atomic ratio of at most 50 as determined by inductively coupled plasma optical emission spectrometry; with preference, the process is according to the first aspect.

### Description of the figures

- Figure 1 represents an overview of a possible process scheme according to the disclosure.
- Figure 2 is a SEM image of SSZ-13 zeolite (CHA family).

### Definitions

For the disclosure, the following definitions are given:

Zeolite codes (e.g., CHA...) are defined according to the "Atlas of Zeolite Framework Types", 6th revised edition, 2007, Elsevier, to which the present application also refers.

The terms "alkane" or "alkanes" as used herein describe acyclic branched or unbranched hydrocarbons having the general formula $C_nH_{2n+2}$, and therefore consisting entirely of hydrogen atoms and saturated carbon atoms; see e.g., IUPAC. Compendium of Chemical Terminology, 2nd ed. (1997). The term "alkanes" accordingly describes unbranched alkanes ("normal-paraffins" or "n-paraffins" or "n-alkanes") and branched alkanes ("iso-paraffins" or "iso-alkanes") but excludes naphthenes (cycloalkanes).

The term "aromatic hydrocarbons" or "aromatics" relates to cyclically conjugated hydrocarbon with a stability (due to derealization) that is significantly greater than that of a hypothetical localized structure (e.g., Kekule structure). The most common method for determining the aromaticity of a given hydrocarbon is the observation of diatropicity in the $^1$H NMR spectrum.

The terms "olefin" or "alkene" as used herein relate to an unsaturated hydrocarbon compound containing at least one carbon-carbon double bond.

The term "mono-olefin" as used herein relates to an unsaturated hydrocarbon compound containing one single carbon-carbon double bond.

The Si/Al atomic ratio corresponds to the amount of $SiO_2$ divided by the amount of $Al_2O_3$ taking into account the fact there are two atoms of aluminium for one atom of silicon. The silica to alumina ratio (also stated as SAR) corresponds to the amount of $SiO_2$ divided by the amount of $A_2O_3$ notwithstanding the proportion of the Si atoms over the Al atoms in the chemical formula of the molecular sieve. Therefore, the value of the SAR always corresponds to twice the value of the Si/Al atomic ratio. SAR is determined by $NH_3$-Temperature Programmed Desorption.

As used herein, the term "C# hydrocarbons", wherein "#" is a positive integer, is meant to describe all hydrocarbons having # carbon atoms. C# hydrocarbons are sometimes indicated as just C#. Moreover, the term "C#+ hydrocarbons" is meant to describe all hydrocarbon molecules having # or more carbon atoms. Accordingly, the expression "C5+ hydrocarbons" is meant to describe a mixture of hydrocarbons having 5 or more carbon atoms.

The symbol "=" in the term "C#= hydrocarbon" indicates that the hydrocarbon concerned is an olefin or an alkene, the notation "=" symbolizing the carbon-carbon double bond. For instance, "C6=" stands for "C6 olefin", or for olefin comprising 6 carbon atoms.

The term "steam" is used to refer to water in the gas phase, which is formed when water boils.

The term "transition metal" refers to an element whose atom has a partially filled d sub-shell, or which can give rise to cations with an incomplete d sub-shell (IUPAC definition). According to this definition, the transition metals are Sc, Ti, V, Cr, Mn, Fe, Co, Ni, Cu, Zn, Y, Zr, Nb, Mo, Tc, Ru, Rh, Pd, Ag, Cd, La, Hf, Ta, W, Re, Os, Ir, Pt, Au, Hg, Ac, Rf, Db, Sg, Bh, Hs, Mt, Ds, Rg, and Cn. The metals Ga, In, Sn, Tl, Pb and Bi are considered as "post-transition" metal.

The yield to particular chemical compounds is determined as the mathematical product between the selectivity to said particular chemical compounds and the conversion rate of the chemical reaction. The mathematical product is expressed as a percentage.

The terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" also include the term "consisting of".

The recitation of numerical ranges by endpoints includes all integer numbers and, where appropriate, fractions subsumed within that range (e.g. 1 to 5 can include 1, 2, 3, 4, 5 when referring to, for example, a number of elements, and can also include 1.5, 2, 2.75 and 3.80, when referring to, for example, measurements). The recitation of endpoints also includes the recited endpoint values themselves (e.g. from 1.0 to 5.0 includes both 1.0 and 5.0). Any numerical range recited herein is intended to include all sub-ranges subsumed therein.

The particular features, structures, characteristics or embodiments may be combined in any suitable manner, as would be apparent to a person skilled in the art from this disclosure, in one or more embodiments.

## Detailed description of the disclosure

The disclosure provides for a process for converting carbon disulphide into C2-C3 olefins. According to the disclosure, the process for converting carbon disulphide into C2-C3 olefins comprises the following steps:

    a) providing a gaseous feed stream comprising at least 10 vol. % of carbon disulphide;
    b) performing a hydrogenation reaction to obtain a first effluent stream comprising methanethiol ($CH_3SH$) and hydrogen sulphide ($H_2S$);
    c) optionally, performing a pre-treatment of at least a part of the first effluent stream to convert at least a part of the methanethiol ($CH_3SH$) into dimethyl sulphide (DMS) and recovering a second effluent stream containing methanethiol ($CH_3SH$) and dimethyl sulphide (DMS);
    d) optionally, removing of at least a part of $H_2S$ from the first effluent stream and/or the second effluent stream when step (c) is carried out;
    e) providing a catalyst composition;
    f) contacting the first effluent stream and/or the second effluent stream when step (c) is carried out, with said catalyst composition at a temperature ranging from 100°C to 700°C, at a pressure ranging from 0.2 MPa to 20 MPa, to convert at least part of said methanethiol and dimethyl sulphide if any into C2-C3 olefins;

g) recovering a product stream comprising C2-C3 olefins and unconverted methanethiol (CH$_3$SH) and dimethyl sulphide (DMS) if any;

wherein said catalyst composition provided in step e) comprises one or more zeolites with a plurality of pores with a shape of an 8-member ring and in that the one or more zeolites having a Si/Al atomic ratio of at most 50 as determined by inductively coupled plasma optical emission spectrometry.

In the present description, the installation will be described simultaneously with the process. The installation to conduct the process for converting carbon disulphide into C2-C3 olefins according to the process of the first aspect is remarkable in that it comprises:

- a hydrogenation unit 3;
- optionally, a pre-treatment unit 7 downstream of said hydrogenation unit 3;
- a conversion unit 11 downstream of the hydrogenation unit 3 or of the pre-treatment unit 7 when said pre-treatment unit 7 is set up, the conversion unit 11 comprising a reactor with a catalyst composition, the reactor having an inlet and an outlet, wherein the catalyst composition comprises one or more zeolites with a plurality of pores with a shape of an 8-member ring and a Si/Al atomic ratio of at most 50 as determined by inductively coupled plasma optical emission spectrometry;
- optionally, a first desulphurisation unit downstream of said hydrogenation unit 3 and upstream of said conversion unit 11, and/or a second desulphurisation unit downstream of said pre-treatment unit 7 when said pre-treatment unit 7 is set up and upstream of said conversion unit 11;
- a line to convey a gaseous feed stream 1 comprising the carbon disulphide to the hydrogenation unit 3;
- a line to convey a first effluent stream 5 exiting the hydrogenation unit 3 to the inlet of the reactor within the conversion unit 11;
- when the installation comprises a pre-treatment unit 7, a line to convey said first effluent stream 5 exiting the hydrogenation unit 3 to the pre-treatment unit 7 and a line to convey a second effluent stream 9 exiting the pre-treatment unit 7 to the inlet of the reactor within the conversion unit 11;
- a first separation unit 15 placed downstream of the conversion unit 11 and configured to separate the unreacted methanethiol and dimethyl sulphide if any and a line 17 to recycle the unreacted methanethiol and dimethyl sulphide if any at the inlet of the reactor within the conversion unit 11;
- optionally, a second separation unit (not shown) to recover a part of the olefins produced in the reactor and a line to recycle part of the olefins recovered at the inlet of the reactor.

For example, step (f) is performed in fixed bed or in fluidized bed; thus, the reactor in the installation is a fixed bed reactor or a fluidized bed reactor.

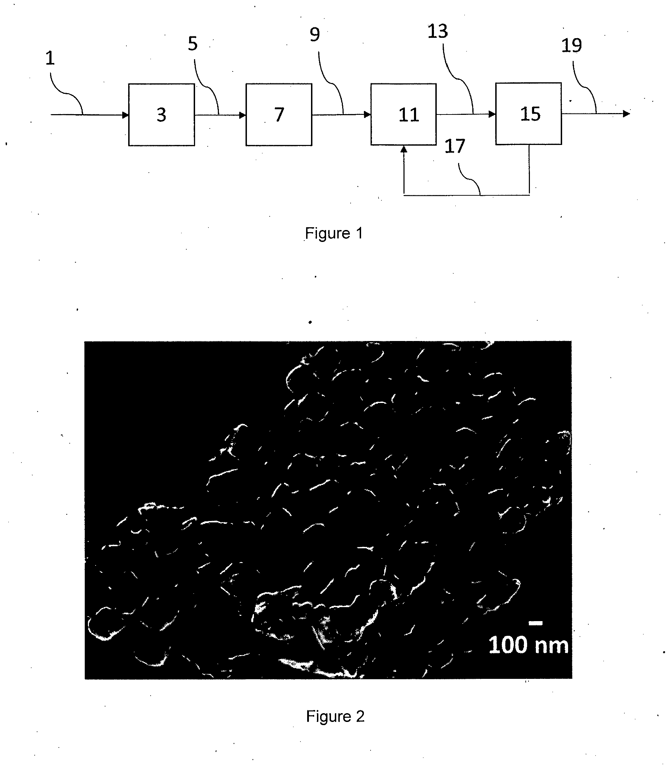

Reference is made to figure 1 wherein a gaseous stream 1 comprising a mixture of CS$_2$ and H$_2$ is sent to a hydrogenation unit 3. Alternatively, the gaseous stream 1 does not comprise H$_2$ and H$_2$ is fed to the hydrogenation unit 3 by another line (not shown). The gaseous stream 1 may contain various components such as H$_2$O, NH3, H$_2$S, inerts and also some traces of hydrocarbons. The first effluent stream 5 containing mainly CH$_3$SH and H$_2$S to be converted can be sent to the pre-treatment unit 7, in which CH$_3$SH can be at least partially converted into DMS, according to the following reactions:

$$2\ CH_3SH \rightarrow H_2S + DMS \qquad (4)$$

The second effluent 9 comprises substantially DMS, CH$_3$SH and H$_2$S and is optionally desulfurized by sweetening or distillation, before being sent into a conversion unit 11. In this conversion unit, CH$_3$SH and DMS are transformed into hydrocarbons (i.e., C2-C3 olefins) and H$_2$S. The product stream 13 is then directed to a first separation unit 15 to separate CH$_3$SH and DMS stream 17 which could be recycled back to the conversion unit 11. The hydrocarbon stream. 19 can thus be directed to a second separation unit (not shown).

The composition of the gaseous feed stream

The gaseous feed stream of step (a) contains carbon disulphide. In an embodiment, the carbon disulphide is present in the gaseous feed stream at a concentration of at least 15 vol % based on the total volume content of the gaseous feed stream; preferably, of at least 25 vol %; most preferably of at least 35 vol%.

In an embodiment, the carbon disulphide is present in the gaseous feed stream at a concentration of 100 vol % based on the total volume content of the gaseous feed stream; preferably, of at most 95 vol %; most preferably of at most 75 vol%.

In a prefered embodiment, said gaseous feed stream of step (a) is diluted with at least one diluent. For example, the gaseous feed stream comprises one or more diluents, wherein the one or more diluents are selected from hydrogen sulfide, steam, methane, ethane, propane, butane, carbon dioxide, carbon monoxide, nitrogen, and any mixture thereof. For example, the one or more diluents are selected from steam, methane, ethane, propane, butane, carbon dioxide, carbon monoxide, nitrogen, and any mixture thereof.

In a preferred embodiment, the one or more diluents are present in the gaseous feed stream at a concentration of at most 90 vol % based on the total volume content of the gaseous feed stream; preferably, of at most 75 vol %; most preferably of at most 65 vol%.

In a most preferred embodiment, the one or more diluents are or comprise methane. With preference, methane is present in said the gaseous feed stream at a concentration ranging from 10 vol % to 90 vol %, preferably at a concentration ranging from 25 vol % to 75 vol %, even more preferably at a concentration ranging from 33 vol % to 66 vol %.

In an embodiment, the one or more diluents comprise $H_2S$ at a concentration of at least 1 vol % to at most 10 vol %.

The step (b) of conversion of the carbon disulphide into methanethiol by hydrogenation reaction

The reaction of hydrogenation of $CS_2$ for production of methyl mercaptan is known to the skilled person and is described for example in international patent application WO2010/046607 and US2017/0158631. This reaction is known to lead to a conversion of $CS_2$ of 100% for a selectivity in terms of methyl mercaptan of 100%, if hydrogen is present at stoichiometry or in excess. The consequence is that the methyl mercaptan produced in this step is very easy to separate from the reaction mixture, since this mixture contains only methyl mercaptan, $H_2S$, and residual amounts of excessive hydrogen:

$$CS_2 + 3\,H_2 \rightarrow CH_3SH + H_2S \qquad (1)$$

Preferentially, this reaction is carried out in the presence of a catalyst, for example a hydrogenation catalyst. It could be selected from those described in international patent application WO2010/046607, in which said hydrogenation catalyst comprises at least one metal doped with at least one alkali metal or alkaline-earth metal hydroxide or oxide.

The metal present in the hydrogenation catalyst may be any metal from group 6 and/or 8 of the Periodic Table of the classification of the Elements (IUPAC), and is preferably selected from the group consisting of nickel (Ni), cobalt (Co), palladium (Pd), rhodium (Rh), platinum (Pt), molybdenum (Mo), tungsten (W), chromium (Cr), iron (Fe) and combinations of two or more of these, preferably combinations of two of these metals, and more particularly Co/Mo, Ni/Mo, Ni/W, W/Mo, with very particular preference being given to the combinations of nickel and molybdenum.

The metal or metals present in the hydrogenation catalyst may also be present directly in the form of metal sulphides. These metal sulphides may also be obtained from the corresponding oxides by any method known to the skilled person.

An example of suitable catalyst, which is sulfided $Mo/Al_2O_3$ catalyst, was described in publication of Oliver Y. Gutiérrez et al. (ChemCatChem, 2011, 3 (9), 1480-1490.

In an embodiment, a suitable hydrogenation catalyst could be an individual compound comprising an alkali metal (A) and a transition metal or a post-transition metal (M), corresponding to the formula $A_xM_yO_nS_n$.

The hydrogenation catalyst is advantageously supported, conventionally, on any type of support generally used within this field, and for example on a support selected from alumina, silica, titanium dioxide ($TiO_2$), zeolites, carbon, zirconium, magnesia (MgO), clays, hydrotalcites and others, and also mixtures of two or more thereof.

In an embodiment, the hydrogenation catalyst is employed in a fixed, fluidized, circulating or ebullated bed.

The reaction is preferably carried out between 100°C and 500°C, and preferably between 200° C and 350°C and under pressure preferably between 0.1 MPa to 30 MPa.

The optional step (c) of conversion of $CH_3SH$ into $CH_3SCH_3$ (DMS)

The step (c) of $CH_3SH$ conversion to DMS serves to adjust the stream composition and reduce the amount of sulfur, as methyl mercaptan could be dimerized according to the following reaction:

$$2CH_3SH \rightarrow CH_3SCH_3 + H_2S \qquad (3)$$

Whereas formed $H_2S$ could be further removed from the stream.

This reaction was described in a publication of E. Huguet et al. (Applied Catalysis B: Environmental, 2013, 134-135, 344-348.

Conversion of methyl mercaptan into DMS is promoted by an acid catalyst, for instance one or more zeolites or alumina. With preference, said one or more zeolites comprise at least one 10-membered ring channel. With preference, the one or more zeolites are selected from the group of MFI, MEL, FER, MTT, MWW, TON, EUO and MRE families, preferably from the MFI family. For example, when the one or more zeolites are selected from the MFI family, the one or more zeolites are selected from ZSM-5, boralite C, silicalite-1, TS-1 and any mixtures thereof. For example, when the one or more zeolites are selected from the MEL family, the one or more zeolites are preferably one or more selected from boralite D, TS-2, ZSM-11, silicalite-2, SSZ-46, and any mixtures thereof. For example, when the one or more zeolites are selected from the FER family, the one or more zeolites are preferably selected from ferrierite, FU-9, ZSM-35, and any mixtures thereof. For example, when the one or more zeolites are selected from the MTT family, the zeolite is preferably ZSM-23. For example, when the one or more zeolites are selected from the MWW family, the one or more zeolites are preferably MCM-22, PSH-3, ITQ-1, MCM-49 and any mixtures thereof. For example, when the one or more zeolites are selected from the TON family, the one or more zeolites are preferably selected from ZSM-22, theta-1, NU-10 and any mixtures thereof. For example, when the one or more zeolites are selected from the EUO family, the one or more zeolites are preferably ZSM-50 and/or EU-1. For example, when the one or more zeolites are selected from the MRE family, the zeolite is preferably ZSM-48.

Alternatively, said catalyst might be an alumina. Examples of suitable materials were described by O. Saur et al., Journal of the Chemical Society, Faraday Transactions 1: Physical Chemistry in Condensed Phases, Volume 1, Issue 77, 1981, 427-437. Non-limiting example of suitable alumina will include $\gamma$-alumina, $\alpha$-alumina, or mixtures thereof.

The catalyst might further comprise a binder, which is selected from silica, clays, calcium phosphates, magnesium phosphates, mullite, and alumina. The binder might be present in an amount of at least 10 wt.% as based on the total weight of the catalyst composition; preferably in an amount of at least 20 wt.%, most preferably in an amount of 30 wt.%, even more preferably in an amount of at least 40 wt.%, and most preferably in an amount of at least 50 wt.%.

The reaction is preferably carried out between 100°C and 400°C, and preferably between 150° C and 250°C and under pressure preferably between 0.1 MPa to 30 MPa.

The optional step (d) of removing of at least a part of $H_2S$ from the first effluent stream and/or the second effluent stream

In an embodiment, step (d) may be conducted when the first effluent stream and/or the second effluent stream that will be used in step (f) contains more than 20 vol.% of $H_2S$ based on the total volume of the first effluent stream and/or the second effluent stream.

When step (d) is conducted on the first effluent stream, step (d) performed in a first desulphurisation unit that is upstream the conversion unit or the pre-treatment unit if step (c) is carried out. When step (d) is conducted on the second effluent stream, step (d) is performed in a second desulphurisation unit that is upstream the conversion unit.

For example, when step (d) of removing of at least a part of $H_2S$ is performed, the stream exiting the desulphurisation unit comprises at most 20 vol.% of $H_2S$ based on the total volume of the stream; preferably, at most 10 vol.%; more preferably at most 5 vol.%.

Removal of excessive hydrogen sulphide from the stream could be done by a fair range of accessible methods: adsorption (by high surface-area carbonaceous or metal oxide sorbents), absorption (with amine or carbonate solvents), membrane removal, distillation, or combination thereof.

The catalyst composition provided in step (e)

According to the disclosure, the catalyst composition provided in step (e) (and used in the conversion step (f)) comprises one or more zeolites with a plurality of pores with a shape of 8-member ring wherein the one or more zeolites have a Si/Al atomic ratio of at most 50 as determined by inductively coupled plasma optical emission spectrometry.

This requirement of having small pores can be understood in that the largest pore has a shape of 8-member ring. For example, a chabazite-type zeolite comprises at least two cages composed of 4- and 8- membered rings connected by one 6-membered double ring.

With regards to the catalyst composition, it can comprise a fairly wide range of zeolites. However, in an embodiment, the one or more zeolites are selected from the group of AEI, AFX, CHA, DDR, ERI, EAB, GIS, KFI, LEV, LTA, RHO, PAU, MWF and RTH families, and any mixture thereof. For example, the one or more zeolites are selected from the group of AEI, CHA, DDR, ERI, KFI, and LEV families, and any mixture thereof. For example, the one or more zeolites are or comprise AEI and/or CHA families.

For example, the one or more zeolites are or comprise SSZ-39 from the AEI family. The synthesis of SSZ-39 is for example disclosed in US10399858.

For example, the one or more zeolites are or comprise SSZ-13 from the CHA family. SSZ-13 zeolites are known to the person skilled in the art. The first synthesis of aluminosilicate CHA zeolite (SSZ-13) has been performed by S.I. Zones in 1985 (see US patent application 4,544,538) using organic-structure-directing agents $N,N,N$-trimethyladamantammonium hydroxide (TMAdaOH) coupled with OH⁻ as a mineralizing agent.

The zeolites can be prepared using an Organic Structure Directing Agents (OSDAs) or without. In some cases, the precursor zeolites contain alkali (e.g., Na⁺, K⁺, Cs+), alkaline earth, or transition metal cations.used, for example, in the preparation of the precursor zeolite. In some embodiments, at least a portion of these cations are still present in as-prepared zeolite.

In some embodiments, the crystalline aluminosilicate compositions of the present disclosure contain an 8-membered ring zeolite structure, and are independently featured by two or more of:

(a) an atomic ratio of tetrahedral to total aluminium atoms in a range having a lower value of about 0.1, 0.12, 0.14, 0.16, 0.18, 0.2, or 0.25, and having an upper value of about 0.2, 0.3, 0.4, 0.5, or 0.6, preferably from about 0.1 to about 0.5, more preferably from about 0.12 to about 0.2;

(b) a microporous region and a mesoporous region, in which the microporous region comprises tetrahedral aluminium and the mesoporous region contains tetrahedral, pentacoordinate, and/or octahedral (hexacoordinate) aluminium;

(c) a micropore volume comprising from 0.03 to 0.15, preferably 0.03 to 0.8, mL/g of the composition.

In certain embodiments, a portion of the aluminium in the zeolite framework may be substituted with a transition metal, boron, gallium, and/or titanium. In certain embodiments, a portion of the silicon in the zeolite framework may be optionally substituted with phosphorus. In certain embodiments, the one or more zeolites could contain tin, molybdenum, tungsten, or iron in the tetrahedral sites of the framework. The one or more zeolites generally may have a significant anionic charge within its framework structure which may be balanced, for example, by cations of elements selected from the group H, Li, Na, K or Cs or the group Mg, Ca, Sr or Ba or the group La or Ce. Although the one or more zeolites may be commonly obtained in a sodium form, a protonic or hydrogen form (via ion-exchange with ammonium hydroxide, and subsequent calcining) is preferred, or a mixed protonic/sodium form may also be used. The one or more zeolites may also be modified by ion-exchange with alkali metal cations, such as Li, K, or Cs, with alkali-earth metal cations, such as Mg, Ca, Sr, or Ba, or with transition metal cations, such as Fe, Ni, Cu, Mn, V, W or with rare-earth metal cations, such as La or Ce. Such subsequent ion-exchange may replace the charge-balancing counter-ions, but furthermore may also partially replace ions in the framework resulting in a modification of the crystalline make-up and structure of the oxide framework.

In an embodiment, the one or more zeolites have a Si/Al atomic ratio ranging from 1 to 50 as determined by inductively coupled plasma optical emission spectrometry (corresponding to a SAR 2 to 100); preferably from 1 to 40; more preferably from 2 to 30; even more preferably from 2 to 25 or from 2 to 17; most preferably from 5 to 20; even mots preferably from 8 to 18; or from 10 to 16; or from 12 to 15.

For example, the one or more zeolites have a Si/Al atomic ratio of at most 45 as determined by inductively coupled plasma optical emission spectrometry; preferably, at most 40; preferably, at most 35; preferably, at most 30; preferably, at most 25; preferably at most 22; more preferably, of at most 20; even more preferably, of at most 18; most preferably, of at most 17; or of at most 16 or of at most 15.

For example, the one or more zeolites have a Si/Al atomic ratio of at least 1 as determined by inductively coupled plasma optical emission spectrometry; preferably at least 2; preferably at least 5; more preferably, of at least 8; even more preferably, of at least 10; most preferably, of at least 11; or of at least 12.

In an embodiment, the step (f) of contacting at least the first effluent stream (i.e., the first effluent or a mixture of the first and second effluent) comprising methanethiol and hydrogen sulphide is carried out with the catalyst composition at a temperature ranging from 100°C to 700°C, at a pressure ranging from 0.2 MPa to 20 MPa, to convert at least part of said methanethiol into C2-C3 olefins, wherein the one or more zeolites of said catalyst composition have a Si/Al atomic ratio of at most 15. This allows enhancing the selectivity to propylene.

In an embodiment, at least one zeolite, or the one or more zeolites contain at least one metal M being a transition metal and/or a post-transition metal with an atomic ratio Si/(Al+ M) ranging from 1 to 50 as determined by inductively coupled plasma optical emission spectrometry; preferably from 1 to 40; more preferably from 2 to 30; even more preferably from 2 to 25 or from 2 to 17; most preferably from 5 to 20; even mots preferably from 8 to 18; or from 10 to 16; or from 12 to 15.

For example, at least one zeolite, or the one or more zeolites contain at least one metal M being a transition metal and/or a post-transition metal with an atomic ratio Si/(Al+ M) of at most 45 as determined by inductively coupled plasma optical emission spectrometry; preferably, at most 40; preferably, at most 35; preferably, at most 30; preferably, at most 25; preferably at most 22; more preferably, of at most 20; even more preferably, of at most 18; most preferably, of at most 17; or of at most 16 or of at most 15.

For example, at least one zeolite, or the one or more zeolites contain at least one metal M being a transition metal and/or a post-transition metal with an atomic ratio Si/(Al+ M) of at least 1 as determined by inductively coupled plasma optical emission spectrometry; preferably at least 2; preferably at least 5; more preferably, of at least 8; even more preferably, of at least 10; most preferably, of at least 11; or of at least 12.

In an embodiment, the step (f) of contacting at least the first effluent stream (i.e., the first effluent or a mixture of the first and second effluent) comprising methanethiol and hydrogen sulphide is carried out with the catalyst composition at a temperature ranging from 100°C to 700°C, at a pressure ranging from 0.2 MPa to 20 MPa, to convert at least part of said methanethiol into C2-C3 olefins, wherein the one or more zeolites of said catalyst composition have a Si/(Al+M) atomic ratio of at most 15. This allows enhancing the selectivity to propylene.

In an embodiment, at least one zeolite, or the one or more zeolites contain at least one metal M being a transition metal and/or a post-transition metal, wherein the transition metal is selected from Fe, W, V, Mo, Zr, Ag, Ni, Cu, Ti, Zn and any mixture thereof and the post-transition metal is selected from Sn and/or In; preferably, the metal M is selected from Fe, W, V, Mo, Sn, Zr, Ag, Ni, Cu, Ti and any mixture thereof; more preferably, the metal M is selected from Fe, W, V, Mo, Sn, Zr, Ag and any mixture thereof; even more preferably, the metal M is selected from Fe, W, V, Mo, Sn, and any mixture thereof; most preferably, the metal M is selected from V, Mo and any mixture thereof. For example, the metal M is Mo.

For example, the one or more zeolites comprise a content ranging from 0.01 to 10.0 wt.% of metal M based on the total mass of the zeolite as measured according to EDS-TEM; preferably a content ranging from 0.1 to 5.0 wt.% more preferably ranging from 0.1 to 1.5 wt.%.

Isomorphous substitution is well known to the person skilled in the art Traditionally, post-synthesis incorporation of heteroelements (for example, a metal M) can be achieved by the treatment of a pre-formed zeolite with a volatile source of a heteroelement at elevated temperature (gas phase isomorphous substitution) or with a solution of the heteroelement source at moderate temperature (hydrothermal isomorphous substitution). Moreover, solid-state reaction can be applied for tailoring the chemical composition of zeolites.

The removal of some framework atoms leading to the formation of silanol groups (Si-OH) usually precedes the insertion of heteroelements. Dealumination, routinely performed by steaming or treatment of zeolites with mineral acids or the combination of both methods, is one of the examples for demetallation treatments.

In contrast, desilication in alkaline medium successfully applied to a large variety of zeolites does not cause significant changes in the Si/Al ratio and, therefore, in acidity. The desilication procedure clearly allows developing mesoporosity in zeolites but quite often at the expense of losing considerable amounts of microporosity. Different variations of the method have been introduced to overpass this drawback, such as the substitution of sodium hydroxide for tetraalkylammonium hydroxide solutions or the application of partial detemplation prior to desilication.

In an embodiment, the one or more zeolites are present in the catalyst composition at a content ranging from 25 wt. % to 95 wt. % based on the total weight of said catalyst composition; preferably ranging from 25 to 90 wt.%; more preferably, ranging from 35 to 88 wt.%; even more preferably ranging from 40 to 85 wt.%; most preferably ranging from 50 to 82 wt.%; even most preferably ranging from 60 to 80 wt.%; for example, ranging from 70 to 85 wt.% or ranging from 50 to 90 wt.%.

The binder is preferably selected from silica, clays, alumina phosphates, calcium phosphates, magnesium phosphates, mullite and any mixture thereof. With preference, the binder is or comprises silica.

The binder is preferably present in an amount of at least 5 wt:% or of at least 10 wt.% as based on the total weight of the catalyst composition, preferably of at least 20 wt.%, most preferably of at least 30 wt.%, even more preferably of at least 40 wt.% and most preferably of at least 50 wt.%.

The binder is preferably present in an amount ranging from 5 wt.% to 75 wt.% based on the total weight of said catalyst composition; preferably ranging from 10 to 75 wt.%; more preferably, ranging from 12 to 65 wt.%; even more preferably ranging from 15 to 60 wt.%; most preferably ranging from 18 to 50 wt.%; even most preferably ranging from 20 to 40 wt.%; for example, ranging from 15 to 30 wt.% or ranging from 10 to 50 wt.%.

For example, the one or more zeolites are shaped with a binder, which is an inorganic material, and preferentially silica or alumina. The one or more zeolites shaped with the binder form a catalyst composition, and the catalyst composition of the present disclosure preferably comprises at least 5 wt.% of a binder, at most 40 wt.% as based on the total weight of the catalyst composition and at most 40 wt.%. Typically, the catalyst composition of the present disclosure comprises between 20 wt.% and 25 wt.% to at most 80 wt.% of a binder as based on the total weight of the catalyst composition.

Non-limiting examples of silicon sources suitable for the binder of the catalyst composition include silicates, precipitated silicas, for example, Zeosil® available from Rhodia, fumed silicas, for example, Aerosil®200 available from Degussa Inc., New York, N.Y., silicon compounds such as tetraalkyl orthosilicates, for example, tetramethyl orthosilicate (TMOS) and tetraethylorthosilicate (TEOS), colloidal silicas or aqueous suspensions thereof, for example, Ludox® HS-40 available from E.I. du Pont de Nemours, Wilmington, Del., silicic acid, alkali-metal silicate, or any combination thereof. Other suitable forms of amorphous silica include silica powders, such as Ultrasil® VN3 SP (commercially available from Degussa). Other non-limiting examples of a suitable solid silica source are special granulated hydrophilic fumed silicas, mesoporous silica and high surface area precipitated silica SIPERNAT® from Evonik, Hi-Sil 233 EP (available from PPG Industries) and Tokusil (available from Tokuyama Asia Pacific).

In addition, suitable amorphous silica sources include silica sols, which are stable colloidal dispersions of amorphous silica particles in an aqueous or organic.liquid medium, preferably water.

Non-limiting examples of commercially available silica sols include those sold under the tradenames Nyacol® (available from Nyacol Nano Technologies, Inc. or PQ Corp.), Nalco (available from Nalco Chemical Company), Ultra-Sol (available from RESI Inc), Ludox® (available from W.R. Grace Davison), NexSil™ (available from NNTI).

Many silica sols are prepared from sodium silicate and inevitably contain sodium. It is, however, found that the presence of sodium ions can cause sintering of the silica body at high temperatures and/or affect catalytic performance. Therefore, if silica sols containing sodium are used, a step of ion exchange may be required in order to reduce or remove sodium. To avoid having out ion exchange steps, it is convenient to use silica sols that contain very little or, ideally, no detectable traces of sodium and have a pH value of less than 7. Most preferably, the silica sol used in the process is slightly acidic with or without polymeric stabilizers. Non-limiting examples of silica sols that contain no detectable traces of sodium include Bindzil® 2034DI, Levasil® 200, Nalco 1034A, Ultra-Sol 7H or NexSil™ 20A.

In some cases, silica dispersion prepared with alkylammonium might be useful. Non-limiting examples of commercially low sodium silica sols stabilized by ammonia or alkylammonium cations include LUDOX® TMA (available from W.R. Grace Davison) or VP WR 8520 from Evonik.

The silica sols with higher $SiO_2$ content than 30 wt.% and even up to 50 wt.%, for example, W1250, W1836, WK341, WK7330 from Evonik are particularly preferred.

The preferred source of silicon is a silica sol or a combination of silica sol with precipitated or fumed silica.

Conversion of the methanethiol and/or the dimethyl sulphide with the catalyst composition

When the catalyst composition is ready, the catalyst is filled in a reactor, which can be a fixed bed, a fluidized bed or another suitable reactor. Preferentially, it can be a fixed-bed tubular reactor.

With preference, the catalyst composition is activated before the step of contacting the feed. The activation is a step of drying/calcination and is performed at high temperatures, preferably between 350 and 850°C. The catalyst composition is preferably calcinated for at least 1 hour, preferentially for at least 6 hours. The one or more zeolites are calcinated before the step of contacting in a nitrogen atmosphere. The step of calcination provides for a crystalline structure to the one or more zeolites.

In a preferred embodiment, a diluent can be added to the gaseous feed stream. Said diluent can be one or more of hydrogen sulphide, steam, C1-C4 alkanes, $CO_2$, $N_2$ or monocyclic aromatics (e.g. benzene, toluene and/or xylene).

With preference, the weight of gaseous feed stream flowing per unit of weight of the catalyst per hour (weight hourly space velocity, WHSV) is comprised ranging from 0.1 $h^{-1}$ to 100 $h^{-1}$, preferably ranging from 0.2 $h^{-1}$ to 50 $h^{-1}$, preferably ranging from 0.3 $h^{-1}$ to 40 $h^{-1}$, preferably ranging from 0.4 $h^{-1}$ to 30 $h^{-1}$, preferably ranging from 0.5 $h^{-1}$ to 20 $h^{-1}$, preferably comprised between 1.0 $h^{-1}$ and 15 $h^{-1}$. Even more preferably, WHSV is ranging from 1.5 $h^{-1}$ to 10 $h^{-1}$. Even more preferably, WHSV is ranging from 2.0 $h^{-1}$ to 6.0 $h^{-1}$. This means that the catalyst of the present disclosure can convert a weight of the feed that is superior to the amount of the catalyst present in the reactor.

In an embodiment, the weight of gaseous feed stream flowing per unit of weight of the catalyst per hour (weight hourly space velocity, WHSV) is comprised ranging from 0.1 $h^{-1}$ to 5.0 $h^{-1}$, preferably ranging from 0.2 $h^{-1}$ to 4.0 $h^{-1}$, preferably ranging from 0.3 $h^{-1}$ to 3.0 $h^{-1}$.

For example, step (f) is performed at a temperature ranging from 200°C to 600°C; preferably ranging from 250°C to 550°C, more preferably, ranging from 300°C to 500°C; even more preferably from 320 °C to 480°C; most preferably to 350 °C to 450°C; and even most preferably from 370°C to 420°C.

For example, step (f) is performed at a temperature of at most 600°C; preferably, of at most 550°C; more preferably, of at most 500°C; even more preferably, of at most 480°C; most preferably, of at most 450°C and even most preferably, of at most 420°C.

For example, step (f) is performed at a temperature of at least 200°C; preferably, of at least 250°C; more preferably, of at least 300°C; even more preferably, of at least 320°C; most preferably, of at least 350°C and even most preferably, of at least 370°C.

For example, step (f) is performed with a partial pressure of carbon disulphide ranging between 10 kPa and 500 kPa, preferentially between 50 kPa and 200 kPa.

The pressure is ranging from 0.2 to 20.0 MPa; preferably ranging from 0.2 and 10.0 MPa, preferably between 0.3 and 5.0 MPa, even more preferably between 0.5 and 2.0 MPa.

In an embodiment, the step (f) of contacting the second effluent with the catalyst composition is followed by a step (g) of recovering a product stream comprising C2-C3 olefins and unconverted methanethiol and dimethyl sulphide if any; wherein the selectivity to C2-C3 of at least 50 %; with preference, of at least 70 %.

A separation step can be foreseen in order to isolate the different components of the product stream. For example, the separation step can be performed by distillation.

The composition of the product obtained after the reaction

The products obtained after step (f) encompass C2-C3 olefins (i.e., ethylene and propylene). However, it is possible to produce low content of other olefins; such as butylenes, preferably but-1-ene, cis- or trans- but-2-ene, or isobutylene, pentenes preferably pent-1-ene, or pent-2-ene.

For example, C2-C3 olefins are ethylene and propylene and/or said C2-C3 olefins are present in said the product stream of step (g) at a concentration of at least 10 vol. %, preferably at least 25 vol. % based on the total molar percent of the product stream recovered at the (g).

In a most preferred embodiment, ethylene and propylene are present in the product stream (g) at a concentration of at least 10 mol %, preferably at least 20 mol % based on the total molar percent of the product stream recovered at step (g).

In a preferred embodiment, the yield of C2-C3 olefins at step (f) is of at least 30 %, preferably 50 % even more preferably at least 60 %.

**Test and determination methods**

The conversion of the one or more alkyl mercaptans $(X_{RSH})$ is determined according to formula (5):

$$X_{total} = \frac{([CH3SH]^i + 2*[CH3SCH3]^i) - ([CH3SH]^f + 2*[CH3SCH3]^f)}{([CH3SH]^i + 2*[CH3SCH3]^i)} x100 \ (5)$$

wherein $[X]^i$ and $[X]^f$ are the molar amount of the compound X in the (initial) feed and the (final) product stream respectively.

The selectivity in methane (C1) is determined according to formula (6):

$$S_{methane} = \frac{[CH_4]}{[CH_4] + 2[C_2H_4] + 2[C_2H_6] + 3[C_3H_6] + 3[C_3H_8] + 4[C_4H_8] + 4[C_4H_{10}] + \cdots} x100 \ (6)$$

wherein the numerator is the carbon adjusted molar amount of methane and the denominator is the sum of all the carbons adjusted molar amount of all hydrocarbons in the product stream.

The selectivity in ethylene (C2=) is determined according to formula (7):

$$S_{ethylene} = \frac{2[C_2H_4]}{[CH_4] + 2[C_2H_4] + 2[C_2H_6] + 3[C_3H_6] + 3[C_3H_8] + 4[C_4H_8] + 4[C_4H_{10}] + \cdots} x100 \ (7)$$

wherein the numerator is the carbon adjusted molar amount of ethylene and the denominator is the sum of all the carbons adjusted molar amount of all hydrocarbons in the product stream.

The selectivity in propylene (C3=) is determined according to formula (8):

$$S_{propylene} = \frac{3[C_3H_6]}{[CH_4] + 2[C_2H_4] + 2[C_2H_6] + 3[C_3H_6] + 3[C_3H_8] + 4[C_4H_8] + 4[C_4H_{10}] + \cdots} x100 \ (8)$$

wherein the numerator is the carbon adjusted molar amount of propylene and the denominator is the sum of all the carbons adjusted molar amount of all hydrocarbons in the product stream.

Gas chromatography experiments were carried out to determine quantitatively the selectivity of the reaction. It was performed on a silica BOND column (60 m x 0.32 mm) using Agilent GC operated by ChemStation software equipped with FID & FPD detectors.

Inductively coupled plasma (ICP) optical emission spectrometry was used to determine the chemical composition of the zeolites using a Varian ICP-OES 720-ES. The Si/Al molar ratio or the Si/(Al+M) molar ratio are determined using the said method.

Nitrogen adsorption was used to determine the surface area of the zeolite.

The adsorption of a nitrogen gas on zeolite can be quantitatively described by an adsorption isotherm, which represents the amount of condensed molecules (the adsorbates) in a porous zeolite (the absorbent) as a function of the partial pressure of the gas phase at a constant temperature. Before the measurements, zeolites were degassed overnight at 300°C. The Brunauer-Emmett-Teller (BET) surface area was calculated by the ASAP 2020 physisorption analyzer built-in software (Micromeritics Corp.). Surface area of the zeolite was calculated using a BET (Brunauer, Emmett, and Teller 1938) single-point method from the measured amount of $N_2$ uptake at liquid nitrogen temperature.

Scanning Electron Microscopy was used to determine the size and shape of the zeolite.

The crystal morphology was studied using a Jeol JS 6701F field emission scanning electron microscope (Jeol Ltd.). The working distance was 8-8.3 mm, the accelerating voltage was 3 kV, probe current was 9.6*10-5 A, emission

current was 10 $\mu$A, extraction voltage was 6.96 kV.

**Examples**

Example 1 - CS$_2$ hydrogenation

The catalyst was prepared from a K$_2$MoO$_4$ (Sigma Aldrich, 98%) deposited on high surface area silica as a carrier. The carrier was impregnated by concentrated solution of K$_2$MoO$_4$ in deionized water, dried at 120°C for 12h and calcined at 550°C for 6h. The loading of K$_2$MoO$_4$ on SiO$_2$ was around 45 wt.% based on the total weight of the catalyst. Prior to the catalytic test, the catalyst was activated by sulfidation in 2 vol.% H$_2$S in Ar at 400°C for 12 h.

For catalytic tests an experimental setup comprising a quartz reactor and GC with PFD detector was utilized. The catalyst sample (30 mL, approximately 25 g) was introduced into the reactor. The test was carried out with gas composition of CS$_2$ 14 vol.%, H$_2$ 43 vol.%, and Ar (balance), temperature was contained at 250°C and pressure of 1.5 bar.

The analysis by gas chromatography of the effluent indicated that the CS$_2$ conversion was 92% with 99% selectivity to methyl mercaptan.

$$X_{CS2} = \frac{[CS2]^i - [CS2]^f}{[CS2]^i} x100$$

Example 2 - CH$_3$SH conversion to DMS

A commercial H-ZSM-5 sample SAR 40 (ChinaCatalyst) with alumina binder was used as a catalyst. Prior to tests, the catalyst was crushed and sieved through 35-45 mesh and activated in nitrogen flow for 12h at temperature of 520°C.

For catalytic tests an experimental setup comprising a quartz reactor and GC with PFD detector was utilized. The catalyst sample (10 mL, approximately 9 g) was introduced to the reactor. The test was carried out with gas composition of CH$_3$SH 5 vol.%, and Ar (balance) at 200°C. The flow rate was adjusted to have WHSV of ~0.3 h-1.

The analysis by gas chromatography of the effluent indicated that the CH$_3$SH conversion was 75% with 100% selectivity to DMS.

$$X_{CH3SH} = \frac{[CH3SH]^i - [CH3SH]^f}{[CH3SH]^i} x100$$

Example 3 conversion into olefins starting from DMS

A catalyst composition comprising SSZ-13 zeolite of the CHA family was prepared using the techniques known in the art (J. Catal. 2013, 298, 27). The as-prepared zeolite had a Si/Al atomic ratio of 15 and a surface area of 826 m$^2$/g, as determined by nitrogen adsorption. The catalyst was further extruded with SiO$_2$ binder, crushed and seized between 35-45 mesh screens and loaded into the reactor. Before the test run it was activated and pre-sulphidized with N$_2$/H$_2$/H$_2$S mixture 96/2/2 mol.%. The reactor was operated at 0.2 MPa and 400°C. A flow of CH$_4$/(CH$_3$)$_2$S ~ 1/1 mol. with WHSV of 2.8 h$^{-1}$ was used. The zeolite was present in the catalyst composition in a content of about 80 wt.% based on the total weight the catalyst composition.

Example 4 - conversion into olefins starting from CH$_3$SH

A catalyst composition comprising SSZ-13 zeolite of the CHA family was prepared using the techniques known in the art (J. Catal. 2013, 298, 27). The as-prepared zeolite had a Si/Al atomic ratio of 15 and a surface area of 826 m$^2$/g, as determined by nitrogen adsorption. The catalyst was further extruded with SiO$_2$ binder, crushed and seized between 35-45 mesh screens and loaded into the reactor. Before the test run it was activated and pre-sulphidized with N$_2$/H$_2$/H$_2$S mixture 96/2/2 mol.%. The reactor was operated at 0.2 MPa and 400°C. The flow of CH$_4$/CH$_3$SH ~ 2/1 mol. with WHSV of 1 h$^{-1}$ was used.

Example 5 - conversion into olefins starting from a mixture of (CH$_3$)$_2$S and CH$_3$SH

A catalyst composition comprising SSZ-13 zeolite of the CHA family was prepared using the techniques known in the art (J. Catal. 2013, 298, 27). The as-prepared zeolite had a Si/Al ratio of 15 and a surface area of 826 $m^2$/g, as determined by nitrogen adsorption. The catalyst was further extruded with $SiO_2$ binder, crushed and seized between 35-45 mesh screens and loaded into the reactor. Before the test run it was pre-activated and pre-sulphidized with $N_2$/$H_2$/$H_2S$ mixture 96/2/2 mol.%. The reactor was operated at 0.2 MPa and 400°C. The flow of $CH_4$/$(CH_3)_2S$/$CH_3SH$ ~ 1/1/2 mol. with WHSV of 2.0 $h^{-1}$ was used.

Table 1: Conversion and selectivity results

|  | Example 1 | Example 2 | Example 3 |
|---|---|---|---|
| Catalyst | Cat. 1 | Cat. 1 | Cat. 1 |
| Temperature (°C) | 400 | 400 | 400 |
| Pressure (MPa) | 0.2 | 0.2 | 0.2 |
| WHSV ($h^{-1}$) | 2.8 | 0.5 | 2.0 |
| **Products** | | | |
| ethane | 2 | 2 | 2 |
| C3 | - | - | - |
| C5= | 5 | 4 | 3 |
| C4= | 4 | 8 | 2 |
| C3= (propylene) | 20 | 54 | 23 |
| C2= (ethylene) | 16 | 24 | 42 |
| **Conversion** | **52** | **90** | **62** |
| **Yield of C2-C3 olefins** | **23.4** | **70.2** | **40.3** |

## Claims

1. Process for converting carbon disulphide into C2-C3 olefins, said process comprising the following steps:

   a) providing a gaseous feed stream (1) comprising at least 10 vol. % of carbon disulphide;
   b) performing a hydrogenation reaction to obtain a first effluent stream (5) comprising methanethiol and hydrogen sulphide;
   c) optionally, performing a pre-treatment of at least a part of the first effluent stream (5) to convert at least a part of the methanethiol into dimethyl sulphide and recovering a second effluent stream (9) containing methanethiol and dimethyl sulphide;
   d) optionally, removing of at least a part of $H_2S$ from the first effluent stream (5) and/or the second effluent stream (9) when step (c) is carried out;
   e) providing a catalyst composition;
   f) contacting the first effluent stream (5) and/or the second effluent stream (9) when step (c) is carried out with said catalyst composition at a temperature ranging from 100°C to 700°C, at a pressure ranging from 0.2 MPa to 20 MPa, to convert at least part of said methanethiol and dimethyl sulphide if any into C2-C3 olefins;
   g) recovering said C2-C3 olefins and unconverted methanethiol and dimethyl sulphide if any;

   **characterized in that** said catalyst composition provided in step e) comprises one or more zeolites with a plurality of pores with a shape of an 8-member ring and **in that** the one or more zeolites having a Si/Al atomic ratio of at most 50 as determined by inductively coupled plasma optical emission spectrometry.

2. The process according to claim 1 is **characterized in that** the one or more zeolites have a Si/Al atomic ratio ranging from 2 to 25 as determined by inductively coupled plasma optical emission spectrometry.

3. The process according to claim 1 or 2 is **characterized in that** the one or more zeolites are selected from the group of AEI, AFX, CHA, DDR, ERI, EAB, GIS, KFI, LEV, LTA, RHO, PAU, MWF and RTH families, and any mixture thereof; preferably, the one or more zeolites are selected from the group of AEI, CHA, DDR, ERI, KFI, and LEV families, and any mixture thereof; more preferably the one or more zeolites are or comprise AEI and/or CHA families.

4. The process according to any one of claims 1 to 3 is **characterized in that** the one or more zeolites are or comprise SSZ-39 from the AEI family.

5. The process according to any one of claims 1 to 4 is **characterized in that** the one or more zeolites are or comprise SSZ-13 from the CHA family.

6. The process according to any one of claims 1 to 5 is **characterized in that** at least one zeolite contains at least one metal M being a transition metal and/or a post-transition metal with an atomic ratio Si/(Al+ M) ranging from 2 to 25 as determined by inductively coupled plasma optical emission spectrometry.

7. The process according to any one of claims 1 to 6 is **characterized in that** at least one zeolite contains at least one metal M being a transition metal and/or a post-transition metal wherein the transition metal is selected from Fe, W, V, Mo, Zr, Ag, Ni, Cu, Ti, Zn and any mixture thereof and the post-transition metal is selected from Sn and/or In.

8. The process according to any one of claims 1 to 7 is **characterized in that** the one or more zeolites are present in the catalyst composition at a content ranging from 25 wt. % to 90 wt. % based on the total weight of said catalyst composition; preferably ranging from 50 to 90 wt.%.

9. The process according to any one of claims 1 to 8 is **characterized in that** the catalyst composition further comprises a binder, wherein said binder is selected from silica, clays, alumina phosphates, calcium phosphates, magnesium phosphates, mullite and any mixture thereof; with preference, the binder is or comprises silica.

10. The process according to any one of claims 1 to 9, **characterized in that** the gaseous feed stream (1) of step (a) contains at least 25 vol. % of carbon disulphide based on the total molar content of the gaseous feed stream (1) and/or **in that** the process further comprises a step of recycling a part of the C2-C3 olefins recovered at step (g) in the first effluent stream (5) and/or in the second effluent stream (9) when step (c) is carried out.

11. The process according to any one of claims 1 to 10, **characterized in that** the gaseous feed stream (1) of step (a) comprises one or more diluents, wherein the one or more diluents are selected from hydrogen sulphide, steam, methane, ethane, propane, butane, carbon dioxide, carbon monoxide, nitrogen, and any mixture thereof; and/or **in that** the gaseous feed stream (1) of step (a) comprises one or more selected from methanethiol; ethanethiol; 1-propanethiol; 2-propanethiol; allyl mercaptan; butanethiol; *tert*-butyl mercaptan; pentanethiols; or any mixture thereof.

12. The process according to any one of claims 1 to 11, **characterized in that** said gaseous feed stream (1) of step (a) further comprises from at least 5 vol. % to at most 25 vol. % of $H_2S$ and/or from 5 vol. % to at most 25 vol. % of COS.

13. The process according to any one of claims 1 to 12, **characterized in that** the step (f) of contacting at least the first effluent stream (5) comprising methanethiol and hydrogen sulphide is carried out with the catalyst composition at a temperature ranging from 100°C to 700°C, at a pressure ranging from 0.2 MPa to 20 MPa, to convert at least part of said methanethiol into C2-C3 olefins, wherein the one or more zeolites of said catalyst composition have a Si/Al atomic ratio of at most 15.

14. Installation to conduct the process for converting carbon disulphide into C2-C3 olefins according to the process defined in any one of claims 1 to 13, the installation is remarkable in that it comprises:

   - a hydrogenation unit (3);
   - optionally, a pre-treatment unit (7) downstream of said hydrogenation unit (3);
   - a conversion unit (11) downstream of the hydrogenation unit (3) or of the pre-treatment unit (7) when said pre-treatment unit (7) is set up, the conversion unit (11) comprising a reactor with a catalyst composition, the reactor having an inlet and an outlet, wherein the catalyst composition comprises one or more zeolites with a plurality of pores with a shape of an 8-member ring and a Si/Al atomic ratio of at most 50 as determined by inductively coupled plasma optical emission spectrometry;
   - optionally, a first desulphurisation unit downstream of said hydrogenation unit (3) and upstream of said conversion unit (11), and/or a second desulphurisation unit downstream of said pre-treatment unit (7) when said pre-treatment unit is set up and upstream of said conversion unit (11);

- a line to convey a gaseous feed stream (1) comprising the carbon disulphide to the hydrogenation unit (3);
- a line to convey a first effluent stream (5) exiting the hydrogenation unit (3) to the inlet of the reactor within the conversion unit (11);
- when the installation comprises a pre-treatment unit (7), a line to convey said first effluent stream (5) exiting the hydrogenation unit (3) to the pre-treatment unit (7) and a line to convey a second effluent stream (9) exiting the pre-treatment unit (7) to the inlet of the reactor within the conversion unit (11);
- a first separation unit (15) placed downstream of the conversion unit (11) and configured to separate the unreacted methanethiol and dimethyl sulphide if any and a line (17) to recycle the unreacted methanethiol and dimethyl sulphide if any at the inlet of the reactor within the conversion unit (11);
- optionally, a second separation unit to recover a part of the olefins produced in the reactor and a line to recycle part of the olefins recovered at the inlet of the reactor.

15. Use of a catalyst composition in a process for converting carbon disulphide into C2-C3 olefins **characterized in that** the catalyst composition comprises one or more zeolites with a plurality of pores with a shape of an 8-member ring and **in that** the one or more zeolites have a Si/Al atomic ratio of at most 50 as determined by inductively coupled plasma optical emission spectrometry; with preference, the process is according to any one of claims 1 to 13.

Figure 1

Figure 2

Europäisches
Patentamt

European
Patent Office

Office européen
des brevets

# EUROPEAN SEARCH REPORT

Application Number

EP 21 31 5225

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | YU, M. ET AL: "Selective methanethiol-to-olefins conversion over HSSZ-13 zeolite", CHEMICAL COMMUNICATIONS, vol. 57, no. 27, 7 April 2021 (2021-04-07), pages 3322-3326, XP002806116, * page 3323 – page 3324, column 2, paragraph 1 * * page 3326, column 1 * | 1-13,15 | INV. C07C1/32 C07C11/04 C07C11/06 B01J32/00 B01J29/70 C07C319/02 |
| Y | WO 2016/001554 A1 (ARKEMA FRANCE [FR]) 7 January 2016 (2016-01-07) * abstract * * step b); claim 1 * | 1-13,15 | |
| X | CA 1 161 775 A (HYDROCARBON RESEARCH INC) 7 February 1984 (1984-02-07) * claim 11 * | 14 | |

TECHNICAL FIELDS
SEARCHED      (IPC)

C07C

The present search report has been drawn up for all claims

| Place of search | Date of completion of the search | Examiner |
|---|---|---|
| The Hague | 4 April 2022 | Delanghe, Patrick |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
...................................................................................................
& : member of the same patent family, corresponding
    document

EPO FORM 1503 03.82 (P04C01)

## EP 4 169 895 A1

This annex lists the patent family members relating to the patent documents cited in the above-mentioned European search report.
The members are as contained in the European Patent Office EDP file on
The European Patent Office is in no way liable for these particulars which are merely given for the purpose of information.

04-04-2022

| Patent document cited in search report | | Publication date | Patent family member(s) | | Publication date |
|---|---|---|---|---|---|
| WO 2016001554 | A1 | 07-01-2016 | AU | 2015282544 A1 | 12-01-2017 |
| | | | BR | 112016030334 B1 | 23-03-2021 |
| | | | CA | 2952754 A1 | 07-01-2016 |
| | | | CN | 107074758 A | 18-08-2017 |
| | | | EA | 201692500 A1 | 28-04-2017 |
| | | | EP | 3164382 A1 | 10-05-2017 |
| | | | ES | 2714904 T3 | 30-05-2019 |
| | | | FR | 3023288 A1 | 08-01-2016 |
| | | | JP | 6698557 B2 | 27-05-2020 |
| | | | JP | 2017519790 A | 20-07-2017 |
| | | | JP | 2019048840 A | 28-03-2019 |
| | | | KR | 20170021863 A | 28-02-2017 |
| | | | KR | 20190011334 A | 01-02-2019 |
| | | | MY | 181193 A | 21-12-2020 |
| | | | NZ | 727771 A | 28-08-2020 |
| | | | PH | 12016502484 A1 | 10-04-2017 |
| | | | PL | 3164382 T3 | 31-07-2019 |
| | | | SG | 11201610786T A | 27-01-2017 |
| | | | TR | 201903045 T4 | 21-03-2019 |
| | | | UA | 119780 C2 | 12-08-2019 |
| | | | US | 2017144966 A1 | 25-05-2017 |
| | | | WO | 2016001554 A1 | 07-01-2016 |
| | | | ZA | 201608778 B | 30-05-2018 |
| CA 1161775 | A | 07-02-1984 | AU | 552164 B2 | 22-05-1986 |
| | | | CA | 1161775 A | 07-02-1984 |
| | | | DE | 3141646 A1 | 10-02-1983 |
| | | | JP | H0525481 A | 02-02-1993 |
| | | | JP | H0559951 B2 | 01-09-1993 |
| | | | JP | H0730341 B2 | 05-04-1995 |
| | | | JP | S57149387 A | 14-09-1982 |

EPO FORM P0459

For more details about this annex : see Official Journal of the European Patent Office, No. 12/82

## REFERENCES CITED IN THE DESCRIPTION

**Patent documents cited in the description**

- US 4543434 A
- US 4822938 A
- US 4864074 A
- US 3488739 A
- US 3880933 A
- WO 2004043883 A
- WO 2010046607 A
- US 20170158631 A
- US 10399858 B
- US 4544538 A, S.I. Zones

**Non-patent literature cited in the description**

- **V. HULEA.** *App. Cat. B.,* 2013, vol. 144, 547-553
- **L. SHUM.** *Int. J. Chem. Kinet.,* 1985, vol. 17, 749-761
- Atlas of Zeolite Framework Types. Elsevier, 2007
- Compendium of Chemical Terminology. 1997
- **OLIVER Y. GUTIÉRREZ et al.** *ChemCatChem,* 2011, vol. 3 (9), 1480-1490
- **E. HUGUET et al.** *Applied Catalysis B: Environmental,* 2013, vol. 134-135, 134-135, 344-348
- **O. SAUR et al.** *Journal of the Chemical Society, Faraday Transactions 1: Physical Chemistry in Condensed Phases,* 1981, vol. 1 (77), 427-437
- *J. Catal.,* 2013, vol. 298, 27